(19) Europäisches Patentamt
European Patent Office
Office européen des brevets

(11) Numéro de publication: **0 024 767**
**B1**

(12) **FASCICULE DE BREVET EUROPEEN**

(45) Date de publication du fascicule du brevet: **01.12.82**

(51) Int. Cl.[3]: **C 07 C 69/753**

(21) Numéro de dépôt: **80200789.8**

(22) Date de dépôt: **22.08.80**

(54) **Diacides carboxyliques chlorobromés, procédé pour leur fabrication et leur utilisation pour rendre des matières synthétiques ignifuges.**

(30) Priorité: **27.08.79 FR 7921629**

(43) Date de publication de la demande: **11.03.81 Bulletin 81/10**

(45) Mention de la délivrance du brevet: **01.12.82 Bulletin 82/48**

(84) Etats contractants désignés: **AT BE CH DE FR GB IT LI NL SE**

(56) Documents cités: **DE-A-1 966 703** **FR-A-1 502 049**

(73) Titulaire: **SOLVAY & Cie (Société Anonyme), Rue du Prince Albert, 33, B-1050 Bruxelles (BE)**

(72) Inventeur: **Paucot, André, Avenue du Général Eisenhower, 63, B-1030 Bruxelles (BE)**

(74) Mandataire: **Elschen, Roland, Solvay & Cie Département de la Propriété Industrielle Rue de Ransbeek 310, B-1120 Bruxelles (BE)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

# Diacides carboxyliques chlorobromés, procédé pour leur fabrication et leur utilisation pour rendre des matières synthétiques ignifuges

La présente invention a pour objet des diacides carboxyliques chlorobromés résultant de la condensation du dibromo-2,3-butène-2-diol-1,4 ou du dibromo-2,3-butanediol-1,4 avec l'acide hexachloroendométhylènetétrahydrophtalique, communément appelé acide chlorendique. Elle concerne également des procédés pour la fabrication de ces produits et leur utilisation en tant qu'additifs, réactifs ou non, pour rendre des matières synthétiques ignifuges.

Il est connu, par les brevets français 1 502 049 et 1 502 050 déposés le 5 octobre 1966 au nom de Produits Chimiques Pechiney-Saint-Gobain, de fabriquer des matières synthétiques ignifuges, telles que des polyesters réticulés ou des polyuréthanes au départ de produits résultant de la polycondensation de mélanges d'acides polycarboxyliques, pouvant être polyhalogénés, avec le dibromo-2,3-butène-2-diol-1,4 ou le dibromo-2,3-butanediol-1,4, mélangé éventuellement à un autre polyol. Les diols et acides polycarboxyliques sont mis en œuvre en proportions telles que le polycondensat obtenu présente un excès de fonctions alcooliques par rapport aux fonctions carboxyliques. Les polycondensats ainsi obtenus ont un inconvénient sérieux. En effet, ils sont constitués non pas d'un produit unique, de formule bien définie, mais d'un mélange de produits dont la composition et la nature des constituants sont variables en fonction des conditions opératoires utilisées et notamment de l'excès de diol et sont très difficilement reproductibles. De ce fait, ces mélanges de polycondensats ne sont pas utilisables en pratique comme agents ignifugeants.

La présente invention vise à fournir des diacides carboxyliques chlorobromés résultant de la condensation du dibromo-2,3-butène-2-5-diol-1,4 ou du dibromo-2,3-butanediol-1,4 et d'un diacide carboxylique polyhalogéné qui ne présentent plus cet inconvénient.

L'invention concerne à cet effet des diacides carboxyliques chlorobromés répondant à la formule

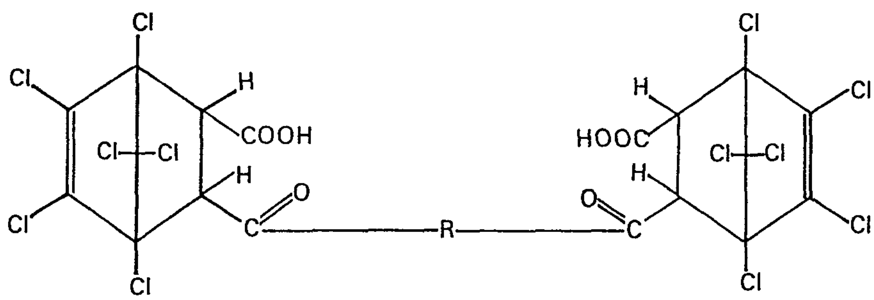

(I)

dans laquelle R représente le radical

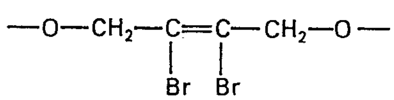

(II)

ou le radical

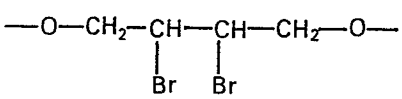

(III)

Le diacide préféré selon l'invention est celui dans lequel R représente le radical

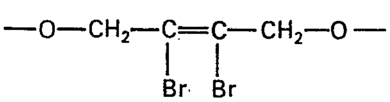

(II)

Le point de fusion de ce produit mesuré sous pression atmosphérique est compris entre 221°C et 224°C.

L'invention concerne également un procédé pour la fabrication de diacides de formule (I).

Selon l'art antérieur, la réaction entre les dibromo-2,3-butène-2-diol-1,4 ou dibromo-2,3-butanediol-1,4 et les acides polycarboxyliques dans des quantités relatives telles qu'il y a un excès de fonctions hydroxyles mène à des mélanges de polycondensats dont il ne serait pas économique de séparer le dérivé diestérifié, si celui-ci était formé.

Le procédé selon l'invention permet d'obtenir les diacides de formule (I) substantiellement purs avec des rendements dépassant 75% par rapport à la quantité de diol mis en œuvre.

Le procédé selon l'invention consiste à faire réagir du dibromo-2,3-butène-2-diol-1,4 ou du dibromo-2,3-butanediol-1,4 avec un excès d'acide chlorendique.

Habituellement, les quantités d'acide chlorendique et de diol mises en œuvre sont telles que le rapport d'acide chlorendique sur diol est supérieur à 2,2 mol par mol. De préférence, on choisit ce rapport supérieur à 2,5 et particulièrement les rapports préférés sont des rapports supérieurs à 3. En général, pour des raisons économiques, on n'utilise pas des rapports molaires supérieurs à 30.

Les autres paramètres de la réaction entre l'acide chlorendique et le diol ne sont pas critiques en eux-mêmes et sont choisis comme habituellement pour ce type de réaction. La température réactionnelle est d'habitude supérieure à 70°C. De préférence, on travaille à des températures comprises entre 80 et 300°C et plus paticu-

lièrement entre 90 et 180°C. Les pressions sont habituellement choisies entre 0,1 et 50 bar. De préférence, on opère entre 0,2 et 10 bar et plus particulièrement à la pression atmosphérique.

La réaction entre l'acide chlorendique et le diol est effectuée en général en milieu liquide. De préférence, ce milieu liquide contient outre les réactifs, un solvant ou un mélange de solvants inertes dans les conditions de la réaction. D'habitude, on met en œuvre des solvants organiques parmi lesquels on préfère ceux dont le point d'ébullition est situé au-dessus de 70°C et plus particulièrement entre 90 et 180°C. Parmi ceux-ci, on préfère les solvants aliphatiques halogénés et les solvants aromatiques substitués ou non. La catégorie de solvants la plus préférée est celle des composés aromatiques ou alkylaromatiques non substitués tels que le benzène, le toluène, les xylènes et leurs homologues supérieurs. De très bons résultats ont été obtenus avec le toluène.

La réaction de diestérification étant une réaction dans laquelle il y a formation d'eau, toute mesure permettant d'éliminer l'eau de réaction peut favoriser la formation des produits de l'invention. Un mode de réalisation préféré du procédé selon l'invention consiste à éliminer l'eau pendant la réaction par évaporation. Un autre consiste à entraîner l'eau par solvant par distillation. Dans ce cas, le solvant peut être avantageusement le toluène parce qu'il forme un azéotrope avec l'eau qui peut être éliminé dans les conditions opératoires habituelles de la réaction au fur et à mesure que la réaction avance.

L'ordre de mise en œuvre de l'acide chlorendique et du diol lors de la réaction n'est pas critique.

Lorsque le procédé est exécuté en milieu solvant, la quantité totale d'acide chlorendique et de diol mise en œuvre est choisie habituellement entre 0,1 et 10% en poids de la masse réactionnelle totale. De préférence, cette quantité est choisie entre 0,2 et 5% en poids.

Dans le but d'accélérer la réaction d'estérification, on peut ajouter au mélange réactionnel un catalyseur du type acide ou basique ainsi que des composés connus pour leurs propriétés de capter l'eau. Des catalyseurs avantageusement utilisés sont les acides de Lewis ou de Brönsted et plus particulièrement les acides protoniques. Parmi ceux-ci, les acides préférés sont les acides forts tels que le chlorure d'hydrogène ou l'acide sulfurique et leurs dérivés. Tout particulièrement, on préfère travailler avec les dérivés de l'acide sulfurique et parmi ceux-ci le catalyseur préféré est l'acide para-toluène sulfonique.

La quantité de catalyseur mise en œuvre est habituellement comprise entre 0,1 et 5%, en poids, calculé par rapport au diol. La quantité préférée en catalyseur se situe entre 0,2 et 1% en poids de diol.

Une fois la réaction d'estérification terminée, on peut séparer facilement le diester formé des réactifs résiduaires et du solvant de la réaction. Pour ce faire, on peut utiliser tout moyen physique ou chimique connu. De préférence, on évapore d'abord tout le solvant éventuel et on extrait ensuite l'acide chlorendique et les diols résiduaires avec de l'eau chaude dans laquelle tous les réactifs non convertis sont solubles. Le solide résiduaire est substantiellement constitué de diacide de formule (I).

On choisit la température de l'eau d'extraction habituellement entre 60 et 100°C et de préférence entre 80 et 95°C. Le diacide qui n'est pas soluble dans l'eau chaude, est obtenu sous forme d'un précipité solide qui peut être séché facilement.

Le procédé de fabrication de diacides de formule (I) peut être appliqué à un mélange de dibromo-2,3-butène-2-diol-1,4 et de dibromo-2,3-butanediol-1,4. On préfère cependant ne mettre en œuvre qu'un seul diol et plus particulièrement le dibromo-2,3-butène-2-diol-1,4.

La présente invention concerne également un procédé pour l'utilisation de diacides de formule (I) en tant qu'additifs pour rendre des matières synthétiques ignifuges.

Ainsi, les diacides de formule (I) peuvent être utilisés en tant qu'additifs non réactifs dans la plupart des matières synthétiques nécessitant une amélioration de leurs propriétés ignifugeantes. Cette possibilité résulte de l'ensemble des propriétés physiques et chimiques de ces diacides qui sont telles que leur migration reste minimale même dans des conditions extrêmes d'utilisation des matières synthétiques. A titre d'exemples de matières synthétiques dans lesquels on peut ajouter avantageusement les diacides, on peut citer les résines polyoléfiniques telles que le polyéthylène, le polypropylène, les résines vinyliques telles que le polychlorure de vinyle, les résines acryliques telles que le polyméthacrylate de méthyle, les résines styréniques telle que le polystyrène, les polyamides, et les polyesters saturés tels que le polytéréphtalate d'éthylèneglycol.

Les diacides de formule (I) peuvent également être utilisés en tant qu'additifs ignifugeants réactifs participant à la constitution de la molécule du composé de base de matière synthétique à l'intervention des groupes carboxyliques. Pour cette utilisation, qui est préférée, les diacides de formule (I) exercent la fonction d'un monomère ou d'un prépolymère, contenant une quantité élevée de chlore et de brome, qui peut participer à toutes les réactions de polycondensation faisant intervenir habituellement des monomères ou prépolymères carboxyliques. Ainsi les diacides de formule (I) peuvent être mis en œuvre pour fabriquer des polyesters, des polyéthers, des polyuréthanes, des polyisocyanurates et d'autres polymères analogues présentant une résistance accrue au feu et à la chaleur et parfaitement reproductibles.

Les exemples ci-après sont donnés pour illustrer l'invention.

Exemple 1

Dans un ballon de 2 l équipé d'un condensateur, on introduit 1,5 l de toluène, 38,8 g (0,100 mol) d'acide chlorendique, 6,15 g (0,025 mol) de

dibromo-2,3-butène-2-diol-1,4 et 0,2 g d'acide para-toluène sulfonique. Le mélange réactionnel est ensuite chauffé à reflux pendant 24 h en éliminant en continu l'eau formée sous forme d'un azéotrope avec le toluène.

Le mélange est ensuite refroidi et lavé avec de l'eau froide. Le toluène de la phase organique est ensuite éliminé par évaporation à sec et on obtient 39 g de résidu solide.

On soumet 32 g de résidu solide à l'extraction par l'eau chaude à 90°C dans un appareil de Soxhlet pendant 48 h afin d'éliminer l'acide chlorendique et le dibromo-2,3-butène-2-diol-1,4 n'ayant pas réagi. Le produit résiduaire est séché à l'étuve à 200°C jusqu'à poids constant qui est de 15,5 g.

Des analyses physiques de ce produit, il ressort qu'il commence à fondre à 211°C et que la fin de la fusion est située à 223,5°C. L'analyse par résonance paramagnétique nucléaire de l'ester méthylique du produit obtenu permet de conclure qu'il s'agit du diacide de formule

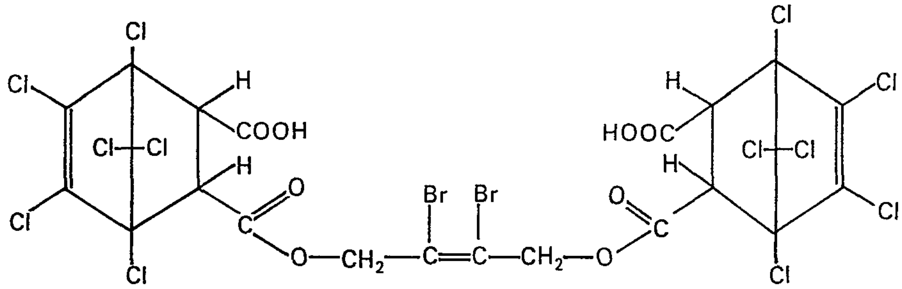

(I)

Ceci est également confirmé par la mesure du poids moléculaire par ébullioscopie dans le tétrahydrofuranne qui donne la valeur de 902 alors que la valeur théorique est 988.

Le rendement de la réaction calculé par rapport au diol est, en se basant sur le poids moléculaire théorique, de 76%.

Exemple 2

Cet exemple est donné à titre comparatif.

On opère dans des conditions identiques à celles décrites pour l'exemple 1 mais en partant de 12,3 g (0,050 mol) de dibromo-2,3-butène-2-diol-1,4.

Les analyses par chromatographie effectués sur le produit obtenu montrent qu'il s'agit là d'un mélange de nombreux composés comprenant les réactifs n'ayant pas réagi et plusieurs esters non identifiés.

Exemple 3

Cet exemple est donné également à titre comparatif.

On opère de nouveau dans des conditions décrites pour l'exemple 1 mais en partant de 19,4 g (0,05 mol) d'acide chlorendique et de 24,6 g (0,100 mol) de dibromo-2,3-butène-2-diol-1,4.

Les analyses par chromatographie effectués sur le produit obtenu montrent que l'on obtient une proportion plus grande, qu'à l'exemple 2, d'esters non identifiés.

On peut conclure de la comparaison des exemples 1, 2 et 3 que seul le procédé dans lequel on travaille avec un excès d'acide chlorendique permet l'obtention d'un seul composé substantiellement pur.

**Revendications pour les Etats contractants: BE, CH/LI, DE, FR, GB, IT, NL, SE**

1. Diacides carboxyliques chlorobromés, caractérisés en ce qu'ils répondent à la formule

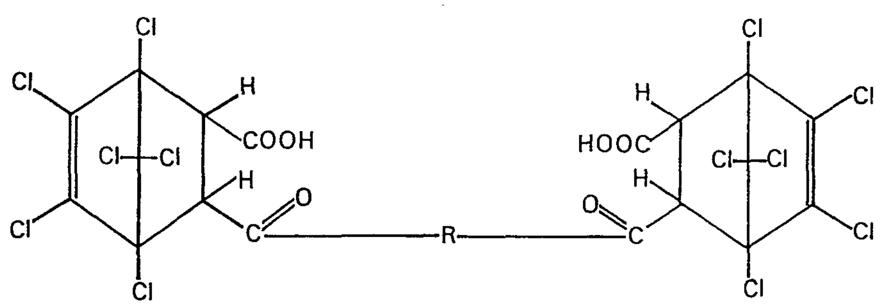

(I)

dans laquelle R représente le radical

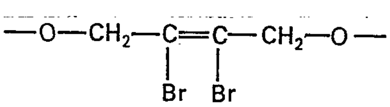

(II)

ou le radical

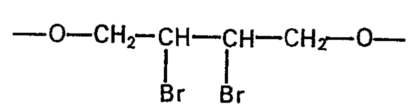

(III)

2. Diacide selon la revendication 1, caractérisé en ce que R représente le radical

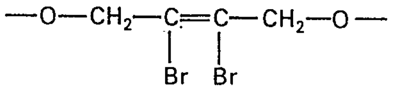
(II)

3. Procédé pour la fabrication de diacides selon la revendication 1 ou 2, selon lequel on fait réagir de l'acide chlorendique et du dibromo-2,3-butène-2-diol-à1,4 ou du dibromo-2,3-butanediol-1,4, caractérisé en ce qu'on met en œuvre un excès d'acide chlorendique.

4. Procédé selon la revendication 3, caractérisé en ce que l'acide chlorendique et le diol sont mis en œuvre dans un rapport supérieur à 2,5 mol d'acide chlorendique par mol de diol.

5. Procédé selon la revendication 3 ou 4, caractérisé en ce que la réaction est réalisée en présence d'un solvant organique.

6. Procédé selon la revendication 5, caractérisé en ce que le solvant organique est le toluène.

7. Procédé selon la revendication 5 ou 6 caractérisé en ce qu'après la réaction, on évapore le solvant organique et on soumet le produit résiduaire à une extraction avec de l'eau se trouvant à une température comprise entre 60 et 100°C.

8. Procédé selon l'une quelconque des revendications 3 à 7, caractérisé en ce que la réaction est réalisée en présence d'un catalyseur choisi parmi les acides forts et leurs dérivés.

9. Procédé pour l'utilisation de diacides selon la revendication 1 ou 2, caractérisé en ce que les diacides sont utilisés pour rendre des matières synthétiques ignifuges.

10. Procédé selon la revendication 9, caractérisé en ce que les diacides sont utilisés en tant que monomères ou prépolymères pour des réactions de polycondensation.

**Revendications pour l'Etat contractant: AT**

1. Procédé pour la fabrication de diacides carboxyliques chlorobromés répondant à la formule

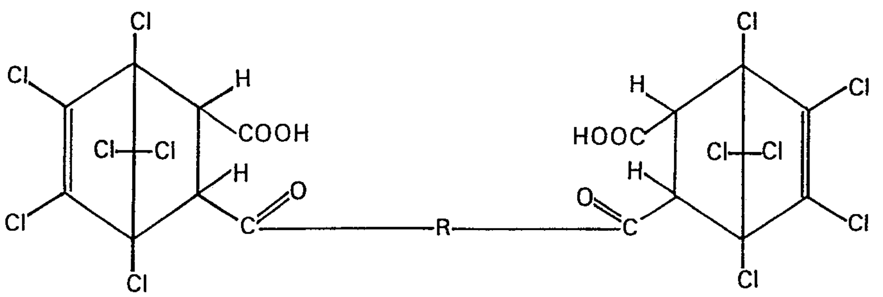
(I)

dans laquelle R représente le radical

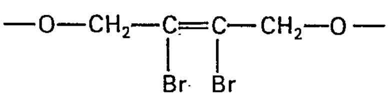
(II)

ou le radical

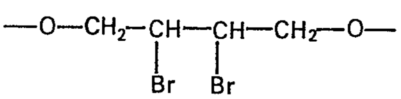
(III)

selon lequel on fait réagir de l'acide chlorendique et respectivement du dibromo-2,3-butène-2-diol-1,4 ou du dibromo-2,3-butanediol-1,4, caractérisé en ce qu'on met en œuvre un excès d'acide chlorendique.

2. Procédé pour la fabrication de diacide selon la revendication 1, caractérisé en ce que R représente le radical

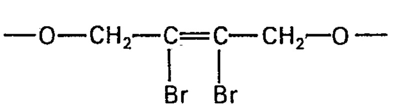
(II)

3. Procédé selon la revendication 1 ou 2, caractérisé en ce que l'acide chlorendique et le diol sont mis en œuvre dans un rapport supérieur à 2,5 mol d'acide chlorendique par mol de diol.

4. Procédé selon l'une quelconque des revendications 1 à 3, caractérisé en ce que la réaction est réalisée en présence d'un solvant organique.

5. Procédé selon la revendication 4, caractérisé en ce que le solvant organique est le toluène.

6. Procédé selon la revendication 4 ou 5, caractérisé en ce qu'après la réaction, on évapore le solvant organique et on soumet le produit résiduaire à une extraction avec de l'eau se trouvant à une température comprise entre 60 et 100°C.

7. Procédé selon l'une quelconque des revendications 1 à 6, caractérisé en ce que la réaction est réalisée en présence d'un catalyseur choisi parmi les acides forts et leurs dérivés.

8. Procédé pour l'utilisation de diacides obtenus selon l'une quelconque des revendications 1 à 7, caractérisé en ce que les diacides sont utilisés pour rendre des matières synthétiques ignifuges.

9. Procédé selon la revendication 8, caractérisé en ce que les diacides sont utilisés en tant que monomères ou prépolymères pour des réactions de polycondensation.

**Patentansprüche für die Vertragsstaaten: BE, CH/LI, DE, FR, GB, IT, NL, SE**

1. Chlorbromierte Dicarbonsäuren, dadurch gekennzeichnet, dass sie der Formel

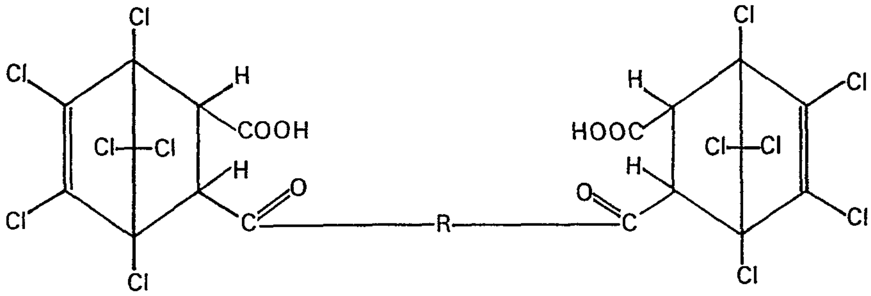

(I)

entsprechen, worin R den Rest

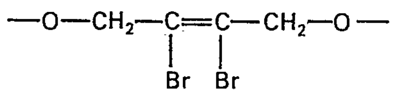

(II)

oder den Rest

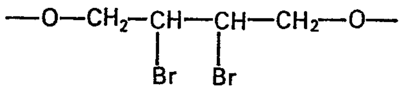

(III)

bedeutet.

2. Disäure gemäss Anspruch 1, dadurch gekennzeichnet, dass R den Rest

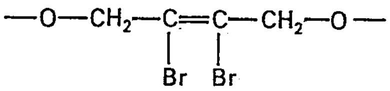

(II)

bedeutet.

3. Verfahren zur Herstellung von Disäuren gemäss Anspruch 1 oder 2, nach dem man Chlorendicsäure und 2,3-Dibrom-2-buten-1,4-diol oder 2,3-Dibrom-butan-1,4-diol reagieren lässt, dadurch gekennzeichent, dass man einen Überschuss an Chlorendicsäure einsetzt.

4. Verfahren nach Anspruch 3, dadurch gekennzeichnet, dass die Chlorendicsäure und das Diol in einem Verhältnis über 2,5 Mol Chlorendicsäure pro Mol Diol eingesetzt werden.

5. Verfahren nach Anspruch 3 oder 4, dadurch gekennzeichnet, dass die Umsetzung in Gegenwart eines organischen Lösungsmittels erfolgt.

6. Verfahren nach Anspruch 5, dadurch gekennzeichnet, dass das organische Lösungsmittel Toluol ist.

7. Verfahren nach Anspruch 5 oder 6, dadurch gekennzeichnet, dass man nach der Umsetzung das organische Lösungsmittel verdampft und das verbleibende Produkt einer Extraktion mit Wasser einer Temperatur zwischen 60 und 100°C unterwirft.

8. Verfahren nach einem der Ansprüche 3 bis 7, dadurch gekenzeichnet, dass die Umsetzung in Gegenwart eines unter starken Säuren und ihren Derivaten ausgewählten Katalysators erfolgt.

9. Verfahren zur Anwendung von Disäuren gemäss Anspruch 1 oder 2, dadurch gekennzeichnet, dass die Disäuren dazu verwendet werden, Kunststoffe feuerfest zu machen.

10. Verfahren nach Anspruch 9, dadurch gekennzeichnet, dass die Disäuren als Monomere oder Vorpolymere für Polykondensationsreaktionen verwendet werden.

**Patentansprüche für den Vertragsstaat: AT**

1. Verfahren zur Herstellung von chlorbromierten Dicarbonsäuren entsprechend der Formel

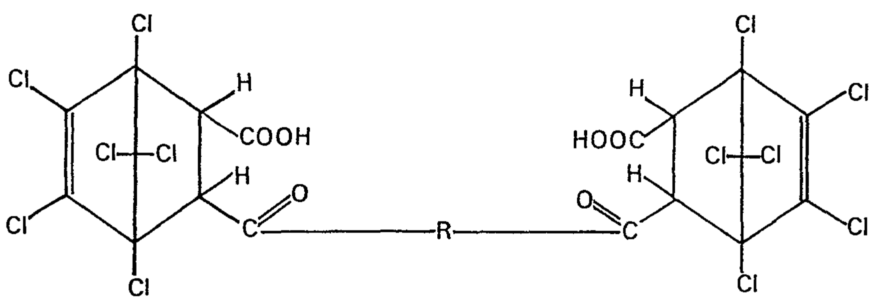

(I)

worin R den Rest

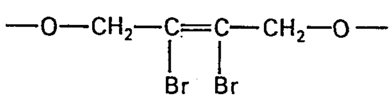

(II)

oder den Rest

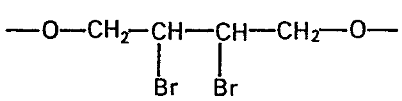

(III)

bedeutet, bei dem man Chlorendicsäure und 2,3-Dibromo-2-buten-1,4-diol oder 2,3-Dibrombutan-1,4-diol reagieren lässt, dadurch gekennzeichnet, dass man einen Überschuss an Chlorendicsäure verwendet.

2. Verfahren zur Herstellung von Disäure gemäss Anspruch 1, dadurch gekennzeichnet, dass R den Rest

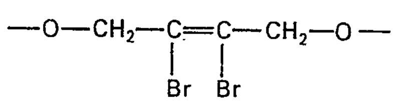

(II)

bedeutet.

3. Verfahren nach Anspruch 1 oder 2, dadurch gekennzeichnet, dass die Chlorendicsäure und das Diol in einem Verhältnis über 2,5 Mol Chlorendicsäure pro Mol Diol eingesetzt werden.

4. Verfahren nach einem der Ansprüche 1 bis 3, dadurch gekennzeichnet, dass die Umsetzung in Gegenwart eines organischen Lösungsmittels erfolgt.

5. Verfahren nach Anspruch 4, dadurch gekennzeichnet, dass das organische Lösungsmittel Toluol ist.

6. Verfahren nach Anspruch 4 oder 5, dadurch gekennzeichnet, dass man nach der Umsetzung das organische Lösungsmittel verdampft und das verbliebene Produkt einer Extraktion mit Wasser einer Temperatur zwischen 60 und 100°C unterwirft.

7. Verfahren nach einem der Ansprüche 1 bis 6, dadurch gekennzeichnet, dass die Umsetzung in Gegenwart eines unter starken Säuren und ihren Derivaten ausgewählten Katalysators erfolgt.

8. Verfahren zur Anwendung von gemäss einem der Ansprüch 1 bis 7 erhaltenen Disäuren, dadurch gekennzeichnet, dass die Disäuren dazu verwendet werden, Kunststoffe feuerfest zu machen.

9. Verfahren nach Anspruch 8, dadurch gekennzeichnet, dass die Disäuren als Monomere oder Vorpolymere für Polykondensationsreaktionen verwendet werden.

**Claims for the contracting states: BE, CH/LI, DE, FR, GB, IT, NL, SE**

1. Chlorobromodicarboxylic acids, characterised in that they correspond to the formula

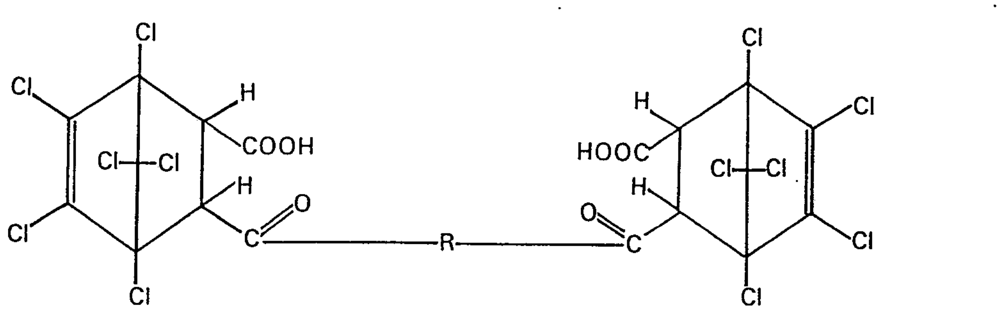

(I)

in which R represents the radical

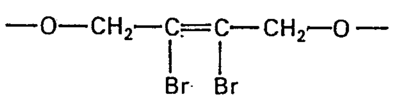

(II)

or the radical

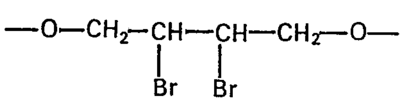

(III)

2. Diacid according to Claim 1, characterised in that R represents the radical

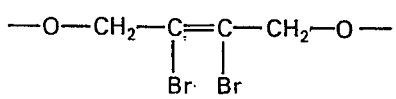

(II)

3. Process for the manufacture of diacids according to Claim 1 or 2, in accordance with which chlorendic acid is reacted with 2,3-dibromobut-2-ene-1,4-diol or 2,3-dibromobutane-1,4-diol, characterised in that an excess of chlorendic acid is used.

4. Process according to Claim 3, characterised in that the chlorendic acid and the diol are used in a ratio of more than 2.5 mols of chlorendic acid per mol of diol.

5. Process according to Claim 3 or 4, characterised in that the reaction is carried out in the presence of an organic solvent.

6. Process according to Claim 5, characterised in that the organic solvent is toluene.

7. Process according to Claim 5 or 6, characterised in that, after the reaction, the organic solvent is evaporated of and the residual product is extracted with water which is at a temperature between 60 and 100°C.

8. Process according to any one of Claims 3 to 7, characterised in that the reaction is carried out in the presence of a catalyst chosen from amongst strong acids and derivatives thereof.

9. Process for using diacids according to Claim 1 or 2, characterised in that the diacids are used to fireproof synthetic materials.

10. Process according to Claim 9, characterised in that the diacids are used as monomers or prepolymers for polycondensation reactions.

**Claims for the contracting state: AT**

1. Process for the preparation of chlorobromodicarboxylic acids corresponding to the formula

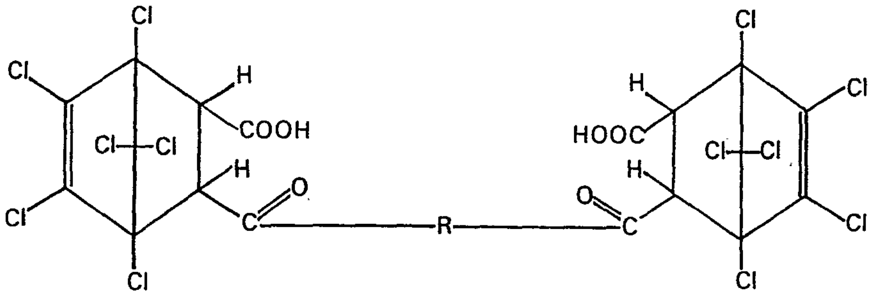

(I)

in which R represents the radical

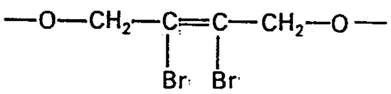

(II)

or the radical

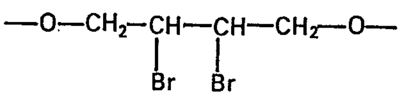

(III)

in accordance with which chlorendic acid is reacted with 2,3-dibromobut-2-ene-1,4-diol or 2,3-dibromobutane-1,4-diol, characterised in that an excess of chlorendic acid is used.

2. Process for the preparation of diacid according to Claim 1, characterised in that R represents the radical

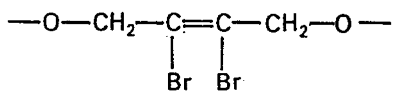

(II)

3. Process according to Claim 1 or 2, characterised in that the chlorendic acid and the diol are used in a ratio of more than 2.5 mols of chlorendic acid per mol of diol.

4. Process according to Claims 1 to 3, characterised in that the reaction is carried out in the presence of an organic solvent.

5. Process according to Claim 4, characterised in that the organic solvent is toluene.

6. Process according to Claim 4 or 5, characterised in that, after the reaction, the organic solvent is evaporated off and the residual product is extracted with water which is at a temperature between 60 and 100°C.

7. Process according to any one of Claims 1 to 6, characterised in that the reaction is carried out in the presence of a catalyst chosen from amongst strong acids and derivatives thereof.

8. Process for using diacids prepared according to Claims 1 to 7, characterised in that the diacids are used to fireproof synthetic materials.

9. Process according to Claim 8, characterised in that the diacids are used as monomers or prepolymers for polycondensation reactions.